# EUROPÄISCHE PATENTSCHRIFT

(11) **EP 4 197 486 B1**
(45) Veröffentlichungstag und Bekanntmachung des Hinweises auf die Patenterteilung: **24.04.2024**
(21) Anmeldenummer: 22170325.9
(22) Anmeldetag: 27.04.2022
(51) Int. Cl.: A61C 13/00, A61C 13/083, A61K 6/818, C04B 35/486, A61K 6/822, A61K 6/824, B32B 18/00, C01G 25/02, A61K 6/807, A61K 6/813, A61C 5/70, A61C 8/00

(54) **ROHLING SOWIE VERFAHREN ZUR HERSTELLUNG EINES SOLCHEN**
BLANK AND METHOD FOR MANUFACTURING THE SAME
BLANC ET PROCÉDÉ DE PRÉPARATION D'UN TEL

(43) Veröffentlichungstag der Anmeldung: 21.06.2023
(73) Patentinhaber: DeguDent GmbH, 63457 Hanau (DE); DENTSPLY SIRONA Inc., York, PA 17401 (US)
(72) Erfinder: VÖLKL, Lothar, 63773 Goldbach (DE); FECHER, Stefan, 63867 Johannesberg (DE); KUTZNER, Martin, 63543 Neuberg (DE); GIESE, Sven, 63452 Hanau (DE); KOHLER, Christian, 63654 Büdingen (DE)
(74) Vertreter: Pichova, Vanda

(56) Entgegenhaltungen:
- EP-A1- 3 517 072
- WO-A1-2014/124874
- CN-A- 113 101 230
- CN-A- 113 105 232
- JP-A- H0 833 701
- US-A1- 2014 265 065

## Beschreibung

Die Erfindung bezieht sich auf einen Rohling zur Verwendung zur Herstellung eines zahntechnischen Produkts, wie Dentalrestauration, Veneer oder Implantatkomponente, bestehend aus oder enthaltend Zirkoniumdioxid (ZrO₂) mit tetragonaler Phase, wobei das Zirkoniumdioxid mit Gadolinium(III)-oxid (Gd₂O₃) als Hauptstabilisator und mit Y₂O₃ als Sekundärstabilisator stabilisiert ist.

Ein mehrphasiger Werkstoff auf Zirkondioxidbasis ist der EP 2 956 427 B1 zu entnehmen. Der Gehalt an tetragonalem Zirkoniumdioxid liegt zwischen 94 Vol.-% und 96 Vol.-%, um die Aufgabe lösen zu können, eine Dentalkeramik mit guten mechanischen Eigenschaften zur Verfügung zu stellen.

Der WO 99/47065 A1 ist ein Verfahren zur Herstellung eines auf einem vorpräparierten Zahnstumpf aufpassbaren Zahnersatz zu entnehmen, dem ein Rohling aus Zirkoniumdioxid zugrunde liegt. Der Rohling besteht aus einer vorgesinterten Zirkoniumdioxidscheibe, aus der eine dem Zahnersatz entsprechende Form herausgearbeitet wird, und zwar unter Berücksichtigung des Schrumpfverhaltens während des Durch- oder Endsinterns. Das Ausgangspulver kann färbende Elemente aufweisen, die in Oxidform vorliegen.

Aus der WO 2005/070322 A1 sind ein anorganisch-anorganischer Kompositwerkstoff und ein Verfahren zu dessen Herstellung bekannt. Zur Herstellung des Kompositwerkstoffes wird aus einem Oxidkeramikpulver aus ZrO₂ (Zirkoniumdioxid) nach formgebender Verarbeitung und Vorsintern ein offenporiges, kristallines Oxid-Keramik-Formteil hergestellt, auf dieses ein Infiltrationsstoff unter Vakuum bei Raumtemperatur aufgebracht und bei Luftatmosphäre und Umgebungsdruck die Oxidkeramik zu dem anorganischanorganischen Kompositwerkstoff verdichtend gesintert. Durch diese Maßnahmen soll sich eine verbesserte ästhetische Wirkung ergeben.

Der WO 2015/199018 A1 ist ein gefärbter transluzenter Zirkoniumdioxidkörper zu entnehmen, der aus yttriumoxidstabilisiertem Zirkoniumdioxid, Erbiumoxid, Eisenoxid, Cobaltoxid und Aluminiumoxid besteht.

Aus der US 8 936 845 B2 ist ein aus Zirkoniumdioxid bestehender Rohling bekannt, der zur Herstellung von Zahnersatz verwendet wird und aus mehreren Schichten unterschiedlicher chemischer Zusammensetzungen besteht. Dabei weisen die einzelnen Schichten unterschiedliche Anteile an Yttriumoxid auf.

Ein aus Zirkoniumdioxid bestehender Rohling zur Herstellung von dentalen Produkten nach der WO 2014/062375 A1 weist zumindest zwei Materialbereiche auf, die unterschiedliche Anteile der tetragonalen und kubischen Kristallphase aufweisen, wobei in einem der Bereiche der Quotient größer und in dem anderen Bereich der Quotient kleiner als 1 ist.

Die EP 2 371 344 A1 bezieht sich auf einen Keramikkörper, der von der Oberfläche ausgehend bis zu einer gewünschten Tiefe mit einem Stabilisierungsmittel angereichert ist.

Der CN 113 101230 A, CN 113 105 232 A und der JP H08 33701 A sind Pulvermischungen für dentale Produkte zu entnehmen, deren Hauptbestandteil ZrO₂ ist. Weitere Bestandteile sind verschiedene Oxide der seltenen Erden, zu denen auch Gd₂O₃ gehört.

Der EP 3 517 072 A1 ist ein Verfahren zur Herstellung von für Dentalrestaurationen bestimmten Rohlingen zu entnehmen. Zur Herstellung werden in eine Matrize Schichten aus Keramikpulver eingefüllt und sodann gepresst, deren Hauptbestandteil Zirkoniumdioxid ist. Dabei weisen die Schichten unterschiedliche Zusammensetzungen auf.

Durch die vorliegende Erfindung soll u.a. die Aufgabe gelöst werden, dass der Rohling die für den Einsatz als dentales Produkt, insbesondere als Restauration oder Implantat, erforderliche Festigkeit aufweist, gleichzeitig gewünschte optische Eigenschaften zeigt, um ästhetischen Anforderungen zu genügen. Dabei soll für die für dentale Restaurationen bestimmten Rohlingen ein Erscheinungsbild gegeben sein, das dem natürlicher Zähne entspricht.

Zur Lösung einer oder mehrerer Aspekte sieht die Erfindung vor, dass das Zirkoniumdioxid mit Gd₂O₃ als Hauptstabilisator mit einem Gehalt zwischen 2,5 bis 4 Mol.-%, relativ zum Gehalt von ZrO₂, sowie mit Y₂O₃ als Sekundärstabilisator mit einem Gehalt zwischen 0,2 und 0,8 Mol.-%, relativ zum Gehalt von ZrO₂, stabilisiert ist und dass die tetragonale Phase des Zirkoniumdioxids zwischen 40 und 80 Vol.-% liegt.

Die vorliegende Erfindung ist in den Patentansprüchen definiert.

Überraschenderweise hat sich gezeigt, dass dann, wenn als Hauptstabilisator Gadolinium(III)-oxid benutzt wird, wodurch eine hohe Bruchzähigkeit bei gleichzeitig guten optischen Eigenschaften erzielbar ist, zumindest ein weiterer Stabilisator als Sekundärstabilisator benutzt wird, die Bruchzähigkeit nicht merklich reduziert wird, gleichzeitig jedoch die Festigkeit erhöht wird. Durch die Erhöhung des Stabilisatorgehalts verbessern sich zudem die optischen Eigenschaften.

Insbesondere ist vorgesehen, dass der Rohling zumindest ein färbendes Oxid aus der Gruppe Pr, Er, Fe, Co, Ni, Ti, V, Cr, Cu, Mn, Tb enthält.

Bevorzugterweise sieht die Erfindung vor, dass der Anteil des Gd₂O₃ zwischen 3 und 4 Mol.-%, liegt, relativ zum Gehalt von ZrO₂.

Die tetragonale Kristallphase des Zirkoniumdioxids des Rohlings liegt bevorzugt zwischen 45 Vol.-% und 75 Vol.-%.

Abweichend von Rohlingen, die als Hauptstabilisator Gd₂O₃ enthalten, wird gezielt der Anteil der tetragonalen Phase des stabilisierten Zirkoniumdioxid herabgesetzt, um einen relativ hohen Anteil von der kubischen Kristallphase zu erhalten, um die gewünschte Transluzenz zu erzielen. Ungeachtet dessen, sind jedoch die erforderliche Festigkeit bzw. Bruchzähigkeit und Biegefestigkeit gegeben, um den mechanischen Anforderungen zu genügen, die an einer Restauration bzw. Implantatkomponente gestellt werden.

Durch den zwingenden Gehalt an zumindest einem färbenden Oxid ist eine optische Erscheinungsform erzielbar, die ästhetischen Anforderungen genügt. Die Färbung bei Implantatkomponenten zeigt den Vorteil, dass dann, wenn durch Veränderungen am Zahnfleisch das Implantat sichtbar wird, dies im Vergleich z.B. zu aus Titan bestehenden Implantaten optisch kaum wahrnehmbar ist.

Der Gehalt des Sekundärstabilisators liegt zwischen 0,2 und 0,8 Mol.-%, relativ zum Gehalt an Zirkoniumdioxid.

Hinsichtlich der zu erzielenden Farbwirkung ist insbesondere vorgesehen, dass der Anteil an dem färbenden Oxid oder den färbenden Oxiden in Gew.-% des Rohlings max. 1,5 beträgt.

Besteht die Möglichkeit, einen einschichtigen und somit einfarbigen Rohling zur Verfügung zu stellen, so ist entsprechend der erfindungsgemäßen Lehre vorgesehen, dass der Rohling zumindest zwei Bereiche mit voneinander abweichenden Zusammensetzungen aufweist.

Dabei kann der Gesamtgehalt des oder der Stabilisatoren in jedem Bereich gleich sein.

Mit anderen Worten kann die Summe der Gehalte der Stabilisatoren in jeder Schicht gleich sein, wobei der Gehalt des oder der färbenden Oxide in den Schichten voneinander abweicht.

In der Erfindung sind s der Gesamtgehalt und/oder die Stabilisatoren in den Bereichen unterschiedlich, wobei Y₂O₃ in den Bereichen vom Gehalt her gleich ist.

Der oder die weiteren Stabilisatoren werden erwähntermaßen als Sekundärstabilisatoren bezeichnet, da der Stabilisator Gd₂O₃ Hauptstabilisator ist, also vorrangig für die Stabilisierung der tetragonalen Kristallphase im Zirkoniumdioxid benutzt wird.

Um gewünschte Fluoreszenzeigenschaften, insbesondere für eine aus dem Rohling herzustellende dentale Restauration, zu erzielen, ist vorgesehen, dass der Rohling oder ein Bereich des Rohlings zumindest ein einen Fluoressenzeffekt erzeugendes Element, insbesondere zumindest ein Oxid aus der Gruppe Bi, Tb, Tm, Pr mit einem Anteil in Gew.-% zwischen 0,005 und 2,0, vorzugsweise zwischen 0,005 und 0,5, enthält.

Um aus einem Rohling eine dentale Restauration, wie Gerüst, Brücke, Krone, Teilkrone, herstellen zu können, die ohne aufwendige Nacharbeiten zur Verfügung gestellt werden kann, gleichzeitig jedoch den gewünschten ästhetischen Anforderungen genügt und in stark belasteten Bereichen die erforderliche Festigkeit aufweist, wird insbesondere vorgeschlagen, dass ein erster Bereich eine Kavität aufweist, innerhalb der sich der eine von dem ersten Bereich abweichende Zusammensetzung aufweisende zweite Bereich erstreckt.

Dabei kann der erste Bereich mehrere Kavitäten, gegebenenfalls unterschiedlicher Innengeometrien, aufweisen, in denen sich mehrere zweite Bereiche erstrecken und dass der erste Bereich eine höhere Transluzenz als der zweite Bereich aufweist, dessen Festigkeit höher als die des ersten Bereichs ist.

Insbesondere ist vorgesehen, dass der Rohling mehrschichtig ausgebildet ist, umfassend zumindest eine unterste und eine oberste Schicht unterschiedlicher Zusammensetzungen, wobei die Schichten zumindest ein erstes färbendes Oxid enthalten, dessen Anteil in der das erste färbende Oxid aufweisenden untersten Schicht geringer als in der das erste färbende Oxid aufweisenden obersten Schicht ist.

Unabhängig hiervon sollte der Rohling zumindest drei Schichten aufweisen, wobei die zwischen einer oberen und einer unteren Schicht verlaufende mittlere Schicht aus einem Material der oberen Schicht und der unteren Schicht besteht.

Um insbesondere ästhetischen Anforderungen zu genügen, sieht ein weiterer Vorschlag der Erfindung vor, dass das erste färbende Oxid zumindest ein Oxid aus der Gruppe Co, Mn, Ni, Cr, insbesondere Co₂O₃ oder MnO₂ oder eine Mischung dieser ist.

Die Erfindung bezieht sich auch auf ein Verfahren zur Herstellung eines Rohlings bestimmt zur Herstellung eines zahntechnischen Produkts, insbesondere dentale Restauration, Veneer, Implantatkomponente, wobei stabilisiertes Zirkoniumdioxid enthaltendes oder aus diesem bestehendes pulverförmiges Material in eine form eingebracht, gepresst und sodann zumindest einer Wärmebehandlung unterzogen wird, wobei das Zirkoniumdioxid mit Gadolinium(III)-oxid (Gd₂O₃) als Hauptstabilisator und Y₂O₃ als Sekundärstabilisator stabilisiert ist und die tetragonale Phase aufweist, das sich dadurch auszeichnet, dass als das keramische Pulver ein solches verwendet wird, dessen Gehalt an Gadolinium(III)-oxid (Gd₂O₃) zwischen 2,5 und 4 Mol.-% und der Gehalt an dem Sekundärstabilisator zwischen 0,2 und 0,8 Mol.-%, jeweils relativ zum Gehalt von ZrO₂ beträgt und die tetragonale Phase des Zirkoniumdioxids zwischen 40 und 80 Vol.-% beträgt.

Vorzugsweise wird dem keramischen Material zumindest ein färbendes Oxid aus der Gruppe Pr, Er, Fe, Co, Ni, Ti, V, Cr, Cu, Mn, Tb zugegeben.

Insbesondere zeichnet sich die Erfindung durch ein Verfahren zur Herstellung eines Rohlings aus einem keramischen Werkstoff aus, wobei in eine Matrize schichtweise zumindest zwei Schichten aus keramischem Material, das aus zuvor beschriebenen Mischungen besteht, wobei die Schichten unterschiedliche Zusammensetzungen aufweisen können, eingebracht und sodann nach Einbringen der Schichten diese gepresst und anschließend gesintert werden, wobei nach Einbringen der ersten Schicht diese oberflächlich derart strukturiert wird, dass die erste Schicht entlang ihrer Oberfläche betrachtet in ihrer Höhe bereichsweise voneinander abweicht, also keine einheitliche Füllhöhe aufweist, und sodann eine von der ersten Schicht in ihrer Zusammensetzung abweichende zweite Schicht in die Form eingebracht wird.

Alternativ besteht die Möglichkeit, dass nach Einbringung der ersten Schicht auf diese eine Zwischenschicht aus einem keramischen Material einer erfindungsgemäßen Mischung in die Matrize eingefüllt wird, das von dem der ersten Schicht abweicht, dass das Material der ersten Schicht mit dem Material der Zwischenschicht vermischt wird, und dass sodann die zweite Schicht in die Matrize eingebracht wird. Dabei ist insbesondere vorgesehen, dass das Material der Zwischenschicht mit dem der ersten Schicht ausgehend von der freien Oberfläche der Zwischenschicht über eine Höhe gemischt wird, die doppelter oder etwa doppelter Höhe der Zwischenschicht entspricht. Ferner ist insbesondere vorgesehen, dass als das Material der Zwischenschicht ein solches verwendet wird, das mit dem der zweiten Schicht übereinstimmt.

Erfindungsgemäß wird nach der ersten Alternative zunächst eine erste Schicht aus schüttfähigem pulverförmigen Material in eine Matrize eingebracht. Nach Einbringen des Materials wird die Oberfläche geglättet, um sodann eine Struktur derart auszubilden, dass sich Erhebungen und Täler ergeben, die insbesondere parallel zueinander, insbesondere jedoch konzentrisch oder parallel zueinander verlaufen. Hierzu ist insbesondere vorgesehen, dass die Struktur durch ein sich relativ zu der ersten Schicht bewegendes, insbesondere drehendes Element gebildet wird, das insbesondere mit einem wellen-, kamm- oder sägezahnartig ausgebildeten Abschnitt die erste Schicht in ihrem Oberflächenbereich strukturiert. Es erfolgt quasi ein "Harken" der Oberfläche, um die Struktur, also die sich abwechselnden Erhebungen und Täler auszubilden.

Insbesondere ist vorgesehen, dass die Struktur derart eingebracht wird, dass Volumen der Erhebungen gleich oder in etwa gleich Volumen der Vertiefungen bzw. Täler ist.

Bevorzugterweise sollte das sägezahnartige Element V-förmige Zähne aufweisen, die symmetrisch ausgebildet sind und deren Flanken einen Winkel zwischen 15° und 45° einschließen. Der Abstand aufeinanderfolgender Zähne, also Abstand Spitze-Spitze sollte zwischen 1 und 4mm, vorzugsweise zwischen 1mm und 3mm liegen.

Sodann wird in die Form das schüttfähige pulverförmige zweite keramische Material eingebracht, das von den durch die Täler gebildeten Senken der Struktur ausgehend mengenmäßig zunimmt, so dass in Folge dessen eine quasi kontinuierliche Zunahme des Anteils der zweiten Schicht über die Höhe der Erhebungen erfolgt. Nachdem die Oberfläche geglättet wurde, werden die Schichten verpresst.

Anschließend erfolgt ein Vorsintern bei einer Temperatur zwischen 700 °C und 1100 °C, insbesondere in einem Bereich zwischen 800 °C und 1000 °C über eine Zeit von z. B. 100 min bis 150 min. Der so hergestellte Rohling wird sodann bearbeitet, um z. B. durch Fräsen und/oder Schleifen eine gewünschte dentale Restauration herzustellen, die anschließend gesintert wird.

Das Sintern erfolgt z. B. über eine Zeit zwischen 10 min und 250 min in einem Temperaturbereich zwischen 1200 °C und 1600 °C.

Insbesondere sollte ein Sintern im Temperaturbereich zwischen 1400 °C und 1500 °C, bevorzugterweise zwischen 1400 °C und 1450 °C, durchgeführt werden.

Die zuvor erläuterten Temperaturen und Zeiten für das Vorsintern bzw. Sintern gelten für unterschiedliche Schichtformen, Schichtfolgen und unterschiedliche Anzahl von Schichten, wobei selbstverständlich dies auch die Herstellung eines Rohlings einschließt, der aus einem einheitlichen Material besteht, also nicht aus Schichten oder Bereichen keramischen Materials besteht, die unterschiedliche Zusammensetzungen bezüglich der Ausgangsrohstoffe aufweisen.

Durch das Durchdringen der Schichten ergibt sich der Vorteil, dass über die Höhe des Rohlings unterschiedliche physikalische und optische Eigenschaften erzielbar sind. So kann dann, wenn die erste Schicht im erforderlichen Umfang eingefärbt ist, nach dem Sintern, also Fertig- oder Durchsintern ein zahnfarbener Randbereich erzielt werden, der über den Übergangsbereich, der durch die sich durchdringenden ersten und zweiten Schichtmaterialien entsteht, in der die Intensität der Zahnfarbe kontinuierlich abnimmt. Aus dem Rohling wird sodann die dentale Restauration insbesondere durch Fräsen unter Berücksichtigung des Schichtverlaufs hergestellt, wobei die dentale Restauration so in den Rohling "genestet" wird, dass im Bereich der zweiten Schicht die Zahnschneide verläuft.

Aufgrund der erfindungsgemäßen Lehre ist ein kontinuierlicher Übergang zwischen den Schichten gegeben, so dass Farbe bzw. Transluzenz kontinuierlich abnimmt bzw. zunimmt.

Durch die Verwendung von Gd₂O₃ als Hauptstabilisator ergibt sich insbesondere der Vorteil, dass eine hohe Kantenstabilität des dentalen Produkts erzielbar ist. Im Vergleich zu mit Y₂O₃ als Hauptstabilisator stabilisiertem ZrO₂ kann die Wandstärke bis zu 20 % dünner sein.

In bevorzugter Weise ist vorgesehen, dass die Möglichkeit des Vermischens der Schichtmaterialien dadurch eröffnet wird, dass ein strukturierendes Element insbesondere um eine entlang der Längsachse der Form verlaufende Achse gedreht wird, um die auch als wellenartige oder sägezahnartige zu bezeichnende Struktur durch Verdrängen von Material der Oberfläche der ersten Schicht zu erzielen. Es besteht auch die Möglichkeit, die Struktur durch ein in Richtung der Oberfläche auf die erste Schicht einwirkendes Druckelement auszubilden, das insbesondere in seiner Oberfläche verlaufende Erhebungen mit zwischen diesen verlaufenden Vertiefungen aufweist, so dass die Negativform von dem auch als Stempel zu bezeichnendem Element in die Oberfläche der ersten Schicht eingeprägt wird. Sodann wird - wie zuvor erläutert - das keramische Material der zweiten Schicht eingefüllt, dann geglättet, um ausschließlich die Schichten gemeinsam zu pressen und den Pressling sodann vorzusintern.

Gegenstand der Erfindung ist auch ein zahntechnisches Produkt in Form einer dentalen Restauration, insbesondere Krone, Teilkrone oder Brücke, wobei die Restauration in Zahnachsenrichtung betrachtet aus zumindest einer wurzelseitig verlaufenden ersten Schicht und einer schneideseitig verlaufenden zweiten Schicht besteht, wobei die Festigkeit der ersten Schicht größer als die der zweiten Schicht und die Transluzenz der zweiten Schicht größer als die der ersten Schicht ist.

Dabei sollten die Schichten ein erstes färbendes Oxid enthalten, insbesondere zumindest ein Oxid aus der Gruppe Co, Mn, Ni, Cr, vorzugsweise Co₃O₄ oder MnO₂ oder eine Mischung dieser, wobei der Anteil in der ersten Schicht geringer als in der zweiten Schicht ist.

Des Weiteren wird vorgeschlagen, dass das zahntechnische Produkt ein einteiliges Implantatsystem bestehend aus Implantat und Abutment ist, wenngleich dies auch in gewohnter Weise zweiteilig sein kann.

Weitere Einzelheiten, Vorteile und Merkmale der Erfindung ergeben sich auch aus der nachfolgenden Beschreibung von den Zeichnungen zu entnehmenden bevorzugten Ausführungsbeispielen sowie deren Erläuterungen.

Es zeigen:

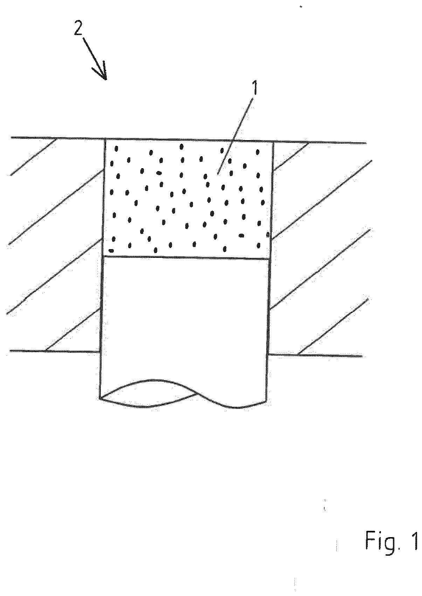

- Fig.1: eine Prinzipdarstellung einer Vorrichtung zur Herstellung eines Rohlings,
- Fig.2: eine Prinzipdarstellung einer Vorrichtung zur Verdeutlichung der Verfahrensschritte zur Herstellung eines Grünlings,
- Fig. 3: einen Rohling mit Bereichen unterschiedlicher Materialeigenschaften,
- Fig. 4: eine weitere Ausführungsform eines Rohlings mit Bereichen unterschiedlicher Materialeigenschaften,
- Fig. 5: eine Prinzipdarstellung eines Rohlings mit aus diesem herauszuarbeitendem Zahnersatz,
- Fig. 6: eine weitere Ausführungsform eines Rohlings mit mehreren Bereichen unterschiedlicher Materialeigenschaften,
- Fig. 7: eine Prinzipdarstellung einer Vorrichtung mit Hilfe dieser durchzuführender Verfahrensschritte zum Herstellen eine Grünlings und
- Fig. 8: eine weitere Prinzipdarstellung zur Erläuterung von Herstellungsverfahrensschritten.

Um einen Rohling herzustellen, wird zunächst ein pulverförmiges keramisches Material hergestellt, dessen Hauptbestandteil stabilisiertes Zirkoniumdioxid ist, das mit Gd₂O₃ als Hauptstabilisator und einem oder mehreren Sekundärstabilisatoren stabilisiert ist.

Der Sekundärstabilisator ist Y₂O₃ mit einem Gehalt zwischen 0,2 und 0,8 Mol.-%, relativ zum Gehalt von ZrO₂.

Der Gesamtgehalt des oder der Stabilisatoren sollte zwischen 2 Mol.-% und 5,8 Mol.-% liegen.

Ferner wird zumindest ein färbendes Oxid hinzugefügt, dessen Gewichtsanteil an dem keramischen Material nicht mehr als 1,5 Gew.-% betragen sollte. Als färbendes Oxid wird insbesondere Fe₂O₃, Er₂O₃, Co₃O₄ oder Tb₂O₃ oder eine Mischung zweier oder mehrerer dieser Oxide gewählt.

Des Weiteren befinden sich in der Mischung in Gew.-% HfO₂ < 3,0, Al₂O₃ < 0,3, technisch bedingte, unvermeidbare Beimengen ≤ 0,2. Ggf. kann ein einen Fluoreszenzeffekt erzeugendes Element wie Bismut oder Thulium beigemischt werden, dessen jeweiliger Oxid-Anteil 0,005 bis 2,0 Gew.-%, insbesondere 0,005 bis 0,5 Gew.-% betragen sollte.

Eine entsprechend hergestellte Mischung 1 wird in eine Form oder Matrize 2 eingefüllt und verpresst. Nach dem Entformen des so hergestellten Grünlings wird dieser sodann einer ersten Wärmebehandlung im Bereich zwischen 800 °C und 1000 °C über einen Zeitraum zwischen 100 min und 150 min unterzogen. Dabei erfolgt zunächst ein Entbindern, sofern der Ausgangsmischung ein Bindemittel zugegeben worden ist, und sodann ein Vorsintern. Der vorgesinterte Rohling wird sodann mechanisch bearbeitet, um z.B. einen künstlichen Zahn herauszuarbeiten. Anschließend erfolgt ein Sintern, also Durchsintern im Temperaturbereich zwischen vorzugsweise 1400 °C und 1500 °C, wobei Werte zwischen 1400 °C und 1450 °C besonders hervorzuheben sind. Der Sinterprozess wird dabei über einen Zeitraum zwischen 10 min und 250 min durchgeführt. Auf diese Weise wird ein monochromatisches dentales Produkt hergestellt. Hierbei kann es sich um eine Dentalrestauration oder z.B. in hervorzuhebender Weise um ein Implantatsystem handeln, das aus einem Implantat und Abutment besteht und somit einstückig hergestellt oder auch -wie üblich- zweiteilig sein kann.

Durch die Verwendung von Gd₂O₃ als Hauptstabilisator ergibt sich der Vorteil, dass abweichend von Vorschlägen aus dem Stand der Technik der Anteil der tetragonalen Kristallphase relativ gering sein kann, und zwar zwischen 40 Vol.-% und 80 Vol.-%, vorzugsweise zwischen 45 Vol.-% und 75 Vol.-%, so dass in Folge dessen der Anteil der kubischen Kristallphase relativ hoch ist und somit eine gewünschte Transluzenz erzielbar ist. Ungeachtet des relativ geringen Anteils an der tetragonale Kristallphase weist ein entsprechender gesinterter Rohling die für einen Einsatz im dentalen Bereich erforderliche Festigkeit, Bruchzähigkeit und Härte auf.

So haben Versuche ergeben, dass die Biaxialfestigkeit gemessen nach ISO 6872 mit Gd₂O₃ stabilisiertem Zirkoniumdioxid, wobei der Gehalt an Gd₂O₃ 3 Mol.-% relativ zum Gehalt vom Zirkoniumdioxid betrug, in Abhängigkeit von Sintertemperaturen im Bereich zwischen 750 MPa bis 1000 MPa und bei mit Gadolinium(III)-oxid als Hauptstabilisator (3 Mol.-%) stabilisiertem Zirkoniumdioxid mit Ytterbiumoxid als Sekundärstabilisator (0,5 Mol.-%) im Bereich zwischen 750 MPa und 850 MPa liegt, Werte, die bei mit Yttriumoxid stabilisiertem ZrO₂ nur dann erreichbar sind, wenn die tetragonale Kristallphase mehr als 90 Vol.-% betrug.

Mit 3 Mol.-% Gd₂O₃ stabilisiertem Zirkoniumdioxid ergab sich eine Bruchzähigkeit Kic, gemessen nach der Chevron-Notch-Beam-Methode, in der Einheit MPa m^{0,5} im Bereich zwischen 10 und 12 bei Sintertemperaturen zwischen 1350 °C und 1450 C°. Bei einer Mischung von Stabilisatoren bestehend aus Gadolinium(III)-oxid als Hauptstabilisator mit 3 Mol.-% und Ytterbiumoxid mit 0,5 Mol.-% als Sekundärstabilisator ergaben sich Kic - Werte zwischen 8 und 10,5 MPa m^{0,5}.

Demgegenüber ergibt sich für Y₂O₃ (3 Mol.-%) stabilisiertes ZrO₂ ein Kic Wert von 4 - 5 MPa m^{0,5}.

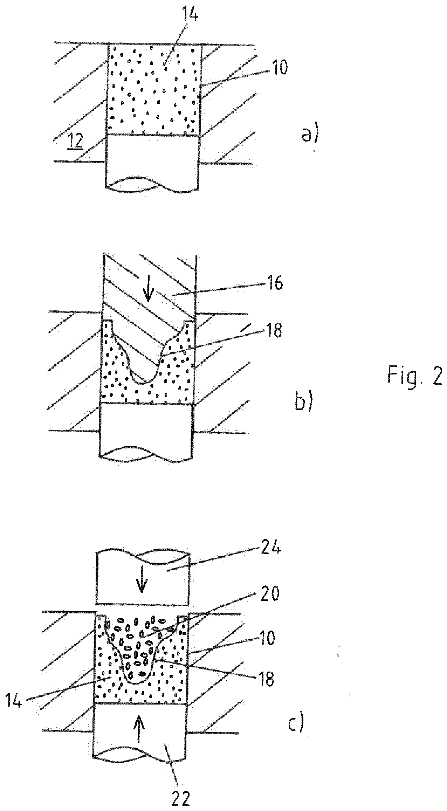

Anhand der Fig. 2 bis 7 sollen weitere die Erfindung mitprägenden Aspekte verdeutlicht werden, um monolithische Produkte, insbesondere dentale Restauration, wie Kronen, Teilkronen oder Brücken herzustellen, wobei die Rohlinge in Bezug auf die herzustellenden Restaurationen konturnahe Bereiche aufweisen.

Es soll erläutert werden, dass Rohlinge herstellbar sind, die Bereiche keramischen Materials mit voneinander abweichenden Zusammensetzungen und damit Eigenschaften aufweisen, um gewünschte optische und mechanische Eigenschaften zu erzielen. Dabei bietet der monolithisch hergestellte Zahnersatz den Vorteil, dass dieser nach dem Herausarbeiten aus dem Rohling und anschließendem Sintern dem Grunde nach unmittelbar verwendet werden kann, ohne dass z.B. eine Schneide händisch aufgebracht und gebrannt werden müsste.

Gezielt können gewünschte Festigkeitswerte eingestellt werden. Gleiches gilt bezüglich farblicher, transluzenter und fluoreszenter Eigenschaften.

Gemäß Fig. 2a wird in eine Matrize 10 ein Pulver einer ersten Zusammensetzung eingefüllt, das z.B. als Schneidematerial benutzt werden soll. Dabei kann das Pulver Bindemittel enthalten. Das Pulver weist eine Zusammensetzung auf, wie dies beispielhaft zuvor erläutert worden ist.

Nach Einfüllen des Materials 14 in die Matrize 10 wird mittels eines Pressstempels 16 eine offene Kavität 18 ausgebildet, wobei das Material selbst dem Grunde nach nicht gepresst wird. Vielmehr wird Material verdrängt bzw. leicht verdichtet. Nach Ausbilden der Kavität (Fig. 2b) wird der Pressstempel 16 entfernt, um in die Kavität ein zweites keramisches Material 20 einzufüllen, das von dem ersten keramischen Material 14 sich dadurch unterscheiden kann, dass der Gehalt der in den Materialien vorhandenen Stabilisatoroxiden in jedem Material gleich ist, jedoch die zugegebenen färbenden Oxide voneinander abweichen, sei es bezüglich des Elements, sei es hinsichtlich des Gewichtsanteils oder einer Kombination dieser.

Gleiches gilt für den Fall, dass Komponenten beigegeben werden, die Fluoreszenzeigenschaften erzielen sollen.

Es besteht jedoch auch die Möglichkeit, dass jedes Material voneinander abweichende Gehalte von Stabilisatoren enthält, wobei jedoch zumindest in jedem Material als Hauptstabilisator Gd₂O₃ und ein weiterer Sekundärstabilisator enthalten ist.

Nach Einfüllen des zweiten keramischen Materials 20 in die Kavität 18 (Fig. 2c) werden sodann die Materialien 14, 20 bzw. die aus diesen gebildeten Schichten oder Bereiche in der Matrize 10 der Presse 12 gepresst und zwar mittels eines Unter- und/oder Oberstempels 22, 24, um ein Verdichten zu erreichen.

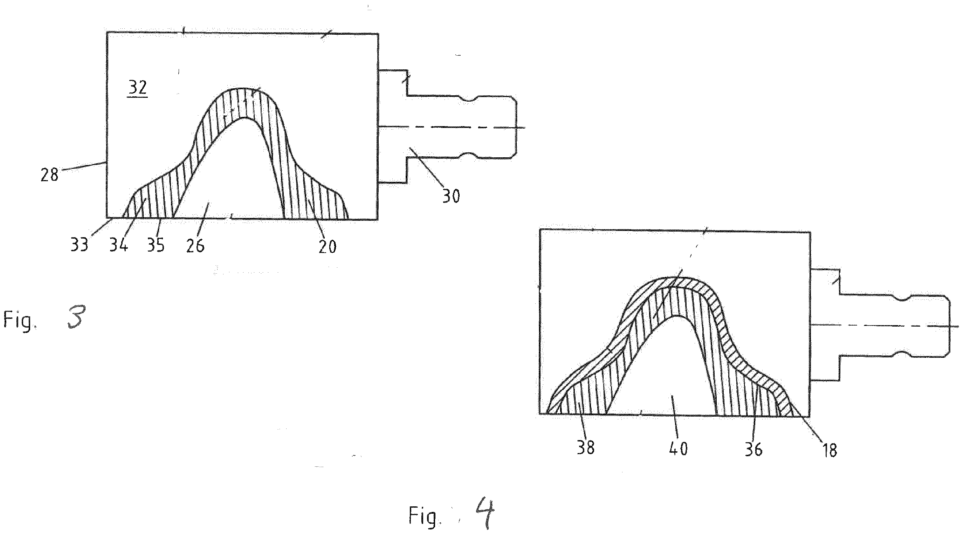

Nach dem Pressen weist der Grünling 28 eine Dichte von in etwa 3g/cm³ auf (Fig. 3). Das Pressen kann in gewünschten üblichen Bereichen mit einem Druck von z.B. zwischen 1000 und 2000 bar durchgeführt werden.

Wie sich aus der Fig. 3 ergibt, kann in das zweite Material 20 nach dessen Verdichten mittels des Pressstempels 22, 24 oder gegebenenfalls nach dem Vorsintern eine zweite Kavität 26 z.B. durch Fräsen herausgearbeitet werden.

Es besteht jedoch auch die Möglichkeit, in das Material 20 gemäß Fig. 2c, das die bodenseitige offene Kavität 18 voll ausfüllt, mittels eines nicht dargestellten Pressstempels eine entsprechende zweite Kavität 26 auszubilden, um ein weiteres Material einzufüllen, das von dem zuvor in die Matrize eingefüllten Material in der Zusammensetzung abweichen sollte.

Unabhängig davon, ob die zweite Kavität 26 vorhanden ist oder nicht, erfolgt nach dem Pressen ein Vorsintern des Rohlings 28, insbesondere im Bereich zwischen 800°C und 1000°C über eine Zeitdauer zwischen 100min und 150min. Dabei erfolgt dann, wenn ein Bindemittel in dem Material vorhanden ist, zunächst ein Entbindern und sodann das Vorsintern.

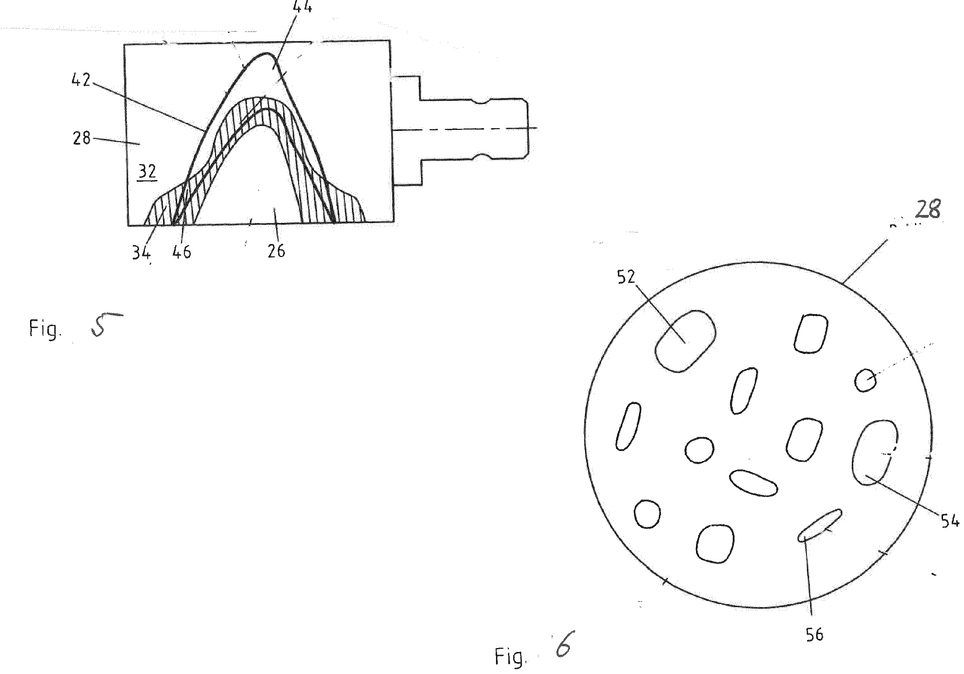

Der Rohling wird mit einer Halterung versehen, um z.B. durch Fräsen und/oder Schleifen eine Bearbeitung durchzuführen, damit aus dem Rohling 28 ein gewünschtes dentales Produkt, wie Zahn, herausgearbeitet werden kann, wie dies anhand der Fig. 5 erläutert werden wird.

Dabei wird bevorzugterweise der herzustellende Zahn zumindest virtuell so in den Rohling 28 gelegt, dass der Schneidebereich in dem von dem ersten keramischen Material 14 gebildeten Bereich 32 und der Dentinbereich abschnittsweise in dem von dem zweiten keramischen Material 20 gebildeten zweiten Bereich 34 verläuft. Sodann erfolgt unter Berücksichtigung dieser Daten ein Bearbeiten des Rohlings 28, wobei das Schrumpfverhalten des Materials Berücksichtigung findet.

Die Fig. 4 verdeutlicht, dass nach Ausfüllen der ersten Kavität 18 in dem ersten keramischen Material 14 und Einfüllen des zweiten keramischen Materials 20 in die Kavität 18 eine zweite Kavität 36 gegebenenfalls entsprechend der Verfahrensweise gemäß Fig. 2b eingebracht wird, um sodann in die so gebildete Kavität 36 ein drittes keramisches Material 38 einzubringen, das von dem zweiten keramischen Material in seiner Zusammensetzung abweicht derart, dass insbesondere eine höhere Festigkeit erzielbar ist.

In dem dritten keramischen Material 38 kann gleichfalls - wie im Zusammenhang mit der Fig. 3 erläutert - eine Kavität 40 ausgebildet werden.

Anhand der Fig. 5 wird verdeutlicht, wie aus dem Rohling 28 eine dentale Restauration, im Ausführungsbeispiel ein Zahn 42, herausgearbeitet wird. Hierzu wird nach Kenntnis des Verlaufs des ersten Bereichs 32 aus dem ersten keramischen Material 14 und dem zweiten Bereich 34 aus dem zweiten keramischen Material 20 in dem Rohling 28 der herzustellende Zahn 42 in die Bereiche 32, 34 virtuell derart gelegt, dass die Schneide im ersten Bereich 32 und das Dentin im zweiten Bereich 34 verläuft.

Nach Herausarbeiten des so virtuell positionierten Zahns 42 aus dem Rohling 28 steht ein Zahnersatz zur Verfügung, der dem Grunde nach unmittelbar einsetzbar ist, insbesondere einer Verblendung nicht bedarf. Ein monolithischer Zahn wird hergestellt. Dabei wird das Herausarbeiten aus dem Rohling dadurch erleichtert, dass der zweite Bereich 34 bereits eine offene Kavität 26 besitzt, wie im Zusammenhang mit der. Fig. 3 erläutert und aus der Fig. 5 ersichtlich wird.

Wie in der Fig. 6 erkennbar, besteht auch die Möglichkeit, einen Rohling 28 mit einer Vielzahl von Bereichen 52, 54, 56 auszubilden, die aus dem zweiten und gegebenenfalls einem dritten keramischen Material bestehen und unterschiedliche Geometrien aufweisen können, um entsprechend Zähne unterschiedlicher Geometrien herstellen zu können. Die aus dem zweiten keramischen Material 20 gebildeten sogenannten zweiten Bereiche 52, 54, 56 sind in dem ersten keramischen Material eingebettet, also von diesem umgeben, wie die Zeichnung verdeutlicht.

Wird bevorzugterweise ein dentales Produkt vor dem Durchsintern des Rohlings aus dem vorgesinterten oder gegebenenfalls aus dem Grünling herausgearbeitet, so wird die Erfindung selbstverständlich dann nicht verlassen, wenn eine diesbezügliche Bearbeitung erst nach dem vollständigen Sintern des Rohlings erfolgt.

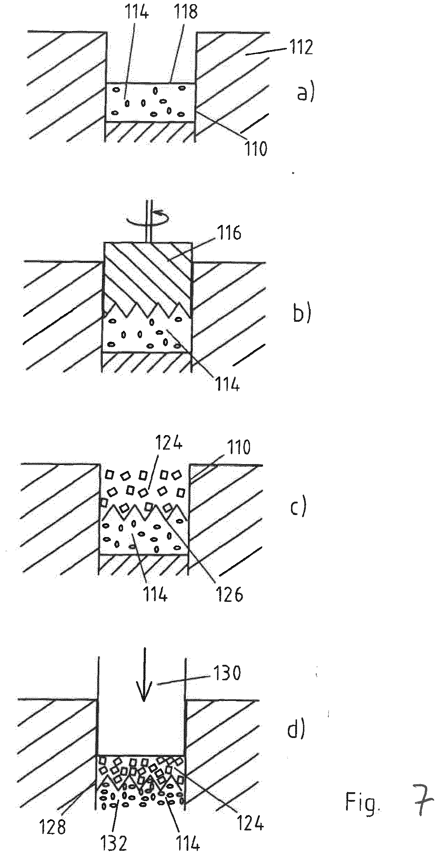

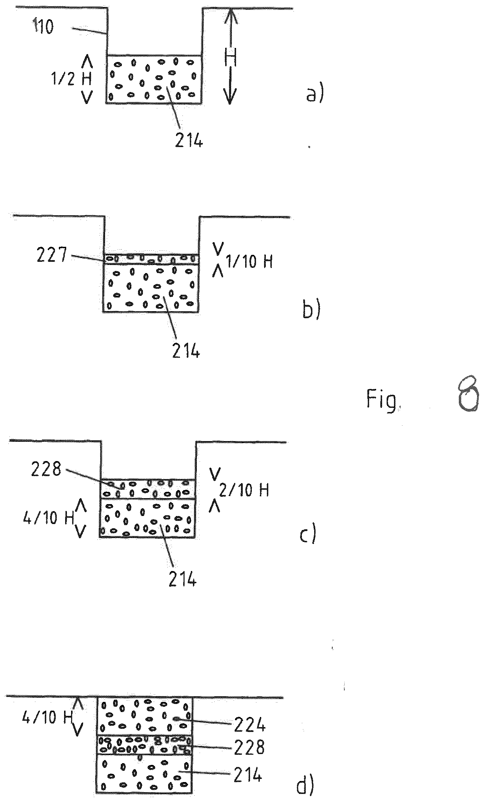

Weitere Ausführungsformen der erfindungsgemäßen Lehre ergeben sich aus den Fig. 7 und 8.

Um einen Rohling herzustellen, der aus Bereichen bzw. Schichten unterschiedlicher Zusammensetzungen besteht, wobei zwischen den Schichten ein stetiger Übergang hinsichtlich der gewünschten Eigenschaften erzielt wird, d.h. z.B. bei einem herzustellenden Zahn ist der Schneidebereich transluzenter als der Dentinbereich und die Festigkeit im Dentinbereich höher als im Schneidebereich, wird in eine Matrize 110 einer Presse 112 ein erstes Material (Schicht 114) eingefüllt, das pulverförmige Ausgangsstoffe enthält, wobei Zirkoniumdioxid das Basismaterial ist, das mit zumindest Gadolinium(III)-oxid als Primärstabilisator und gegebenenfalls mit einem oder mehreren Sekundärstabilisatoren stabilisiert ist. Im erforderlichen Umfang sind ein oder mehrere färbende Oxide und/oder Elemente beigemischt, die Fluoreszenzeigenschaften zeigen. Bindemittel kann gleichfalls zugegeben sein.

Nach Einfüllen des ersten Materials in die Matrize 110 wird die Oberfläche geglättet und gemäß Fig. 7b strukturiert. Hierzu wird ein z.B. scheiben- oder platten- oder stegförmiges Element 116 benutzt, das im Ausführungsbeispiel schichtseitig eine Zackengeometrie aufweist, so dass in der Oberfläche 118 der Schicht 114 eine entsprechende negative Struktur durch Verdrängen von Material ausgebildet wird. Diese Struktur stellt sich als konzentrisch verlaufende Erhebungen und von diesen umgebenen Täler dar. Die Struktur sollte dabei derart ausgebildet sein, dass das Volumen der Erhebungen gleich oder in etwa gleich dem Volumen der Vertiefungen bzw. Tälern ist.

Entsprechend Fig. 7c wird sodann ein aus einem zweiten Material bestehende Schicht 124 in die Matrize 110 eingefüllt, die von dem ersten Material in Bezug auf den Gehalt eines Stabilisators oder der Zusammensetzung der Stabilisatoren oder bei konstantem Gehalt an Stabilisator/en in Bezug auf die weiteren Komponenten voneinander abweichen.

Dadurch, dass das Material der zweiten Schicht 124 bis in den Boden der Täler 126 in der Oberfläche 118 der Schicht eindringt, ergibt sich ein kontinuierlicher Übergang zwischen den Eigenschaften der Schicht 114 und der Schicht 124, nachdem gemäß Fig. 7d die Schichten 124, 114 gepresst worden sind. Die Übergangsschicht ist in der Fig. 7d mit 128 gekennzeichnet.

Erwähntermaßen besteht die Schicht 124 aus einem Material, das von dem der Schicht 114 abweicht. Die Abweichung sollte insbesondere in den Farbzusätzen und dann, wenn ein oder mehrere eine Fluoreszenz erzeugende Elemente beigemischt sind, in deren Anteilen abweichen.

Bezüglich möglicherweise Abweichungen hinsichtlich der Stabilisatoren ist anzumerken, dass die zweite Schicht 124 in Bezug auf den tetragonalen Kristallphasenanteil in der Schicht 124 geringer als in der Schicht 114 sein sollte.

Ein alternatives Verfahren, um einen sogenannten 2D-Rohling herzustellen, also einen solchen, in dem keine Bereiche ausgebildet sind, die konturmäßig an das herzustellende Dentalprodukt angepasst sind, wie dies anhand der Fig. 2 bis 6 erläutert worden ist und daher die Rohlinge als 3D-Rohlinge bezeichnet werden können, ist der Fig. 8 zu entnehmen.

So wird in eine Matrize 110 zunächst ein pulverförmiges erstes keramisches Material eingebracht, das dem der Schicht 114 gemäß Fig. 7 entsprechen kann. Die entsprechende Schicht ist in Fig. 8a mit 214 gekennzeichnet. Die Höhe dieser Schicht 214 kann der halben Höhe der Gesamtschichten betragen, die in die Matrize 110 eingebracht werden.

Auf die Schicht 214 wird sodann eine Schicht 227 mit einer Dicke aufgebracht, die z.B. 1/10 der Gesamthöhe der Schichten ist. Das Material der Schicht 227 kann dem der zweiten Schicht 124 gemäß Fig. 7 entsprechen. Sodann erfolgt ein Vermischen der Schicht 227 mit einem Oberflächenbereich der Schicht 214 über eine Tiefe, die der Dicke der Schicht 227 entsprechen sollte. Hierdurch wird eine Zwischenschicht 228 gebildet, die unter zuvor erfolgter Angaben eine Dicke 2/10 der Gesamthöhe der Schichten aufweist. Auf die Zwischenschicht 228 wird sodann eine weitere Schicht 224 aufgebracht, die der zweiten Schicht 124 gemäß Fig. 7 entspricht.

Die Höhe der Schicht 224 beläuft sich im Ausführungsbeispiel somit auf 4/10 der Gesamthöhe H. Anschließend werden die Schichten 224, 228, 214 insgesamt gepresst, um sodann die Verfahrensschritte Vorsintern, Bearbeiten und Sintern (Durchsintern) durchzuführen, wie dies erläutert worden ist. Ein Bearbeiten nach dem Sintern kann selbstverständlich auch erfolgen.

## Patentansprüche

1. Rohling zur Verwendung zur Herstellung eines zahntechnischen Produkts, wie Dentalrestauration, Veneer oder Implantatkomponente, bestehend aus oder enthaltend Zirkoniumdioxid (ZrO₂) mit tetragonaler Phase, wobei das Zirkoniumdioxid mit Gadolinium(III)-oxid (Gd₂O₃) als Hauptstabilisator und mit Y₂O₃ als Sekundärstabilisator stabilisiert ist,
wobei das Zirkoniumdioxid mit Gd₂O₃ als der Hauptstabilisator, sowie mit dem Sekundärstabilisator mit einem Gehalt zwischen 0,2 und 0,8 Mol.-%, relativ zum Gehalt von ZrO₂, stabilisiert ist und die tetragonale Phase des Zirkoniumdioxids zwischen 40 und 80 Vol.-% liegt,
wobei der Gehalt des Gd₂O₃ zwischen 2,5 und 4 Mol.-%, bevorzugt zwischen 3 und 4 Mol.-%, relativ zum Gehalt von ZrO₂, liegt,
wobei der Rohling zumindest ein färbendes Oxid aus der Gruppe Pr, Er, Fe, Co, Ni, Ti, V, Cr, Cu, Mn, Tb enthält,
wobei der Rohling zumindest zwei Bereiche mit voneinander abweichenden Zusammensetzungen aufweist,
wobei der Gesamtgehalt und/oder die Stabilisatoren in den zumindest zwei Bereichen unterschiedlich sind, wobei Y₂O₃ in den zumindest zwei Bereichen vom Gehalt her gleich ist,
wobei der Gehalt des oder der färbenden Oxide in den zumindest zwei Bereichen voneinander abweicht.

2. Rohling nach Anspruch 1,
**dadurch gekennzeichnet,**
**dass** die tetragonale Phase des Zirkoniumdioxids zwischen 45 Vol.-% und 75 Vol.-% liegt.

3. Rohling nach zumindest einem der vorhergehenden Ansprüche,
**dadurch gekennzeichnet,**
**dass** der Anteil an dem färbenden Oxid oder den färbenden Oxiden in Gew.-% des Rohlings max. 1,5 beträgt.

4. Rohling nach zumindest einem der vorhergehenden Ansprüche,
**dadurch gekennzeichnet,**
**dass** der Rohling oder ein Bereich des Rohlings zumindest ein einen Fluoressenzeffekt erzeugendes Element, insbesondere zumindest ein Oxid aus der Gruppe Bi, Tb, Tm, Pr, mit einem Anteil in Gew.-% zwischen 0,005 und 2,0, vorzugsweise zwischen 0,005 und 0,5, enthält.

5. Rohling nach zumindest einem der vorhergehenden Ansprüche,
**dadurch gekennzeichnet,**
**dass** ein erster Bereich eine Kavität aufweist, innerhalb der sich der eine von dem ersten Bereich abweichende Zusammensetzung aufweisende zweite Bereich erstreckt, wobei gegebenenfalls der erste Bereich mehrere Kavitäten, gegebenenfalls unterschiedlicher Innengeometrie, aufweist, in denen sich mehrere zweite Bereiche erstrecken und dass vorzugsweise der erste Bereich eine höhere Transluzenz als der zweite Bereich aufweist, dessen Festigkeit höher als die des ersten Bereichs ist.

6. Rohling nach zumindest einem der vorhergehenden Ansprüche,
**dadurch gekennzeichnet,**
**dass** der Rohling zumindest drei Schichten aufweist, wobei die zwischen einer oberen und einer unteren Schicht verlaufende mittlere Schicht aus einem Material der oberen Schicht und der unteren Schicht besteht.

7. Rohling nach zumindest einem der vorhergehenden Ansprüche,
**dadurch gekennzeichnet,**
**dass** der Rohling mehrschichtig ausgebildet ist, umfassend zumindest eine unterste Schicht und eine oberste Schicht unterschiedlicher Zusammensetzungen, wobei die Schichten zumindest ein erstes färbendes Oxid aus der Gruppe Co, Mn, Ni, Cr enthalten, dessen Anteil in der das erste färbende Oxid aufweisenden untersten Schicht geringer als in der das erste färbende Oxid aufweisenden obersten Schicht ist, wobei vorzugsweise das erste färbende Oxid zumindest ein Oxid aus der Gruppe Co, Mn, Ni, Cr, insbesondere Co₂O₃ oder MnO₂ oder eine Mischung dieser ist.

8. Verfahren zur Herstellung eines Rohlings bestimmt zur Herstellung eines zahntechnischen Produkts, insbesondere dentale Restauration, Veneer, Implantatkomponente, wobei stabilisiertes Zirkoniumdioxid enthaltendes oder aus diesem bestehendes pulverförmiges Material in eine Form eingebracht, gepresst und sodann zumindest einer Wärmebehandlung unterzogen wird, wobei das Zirkoniumdioxid mit Gadolinium(III)-oxid (Gd₂O₃) als Hauptstabilisator und mit Y₂O₃ als Sekundärstabilisator stabilisiert ist, und die tetragonale Phase aufweist, wobei als das keramische Pulver ein solches verwendet wird, dessen Gehalt an Gadolinium(III)-oxid (Gd₂O₃) zwischen 2,5 und 4 Mol.-% und der Gehalt an dem Sekundärstabilisator zwischen 0,2 und 0,8 Mol.-%, jeweils relativ zum Gehalt von ZrO₂ beträgt und die tetragonale Phase des Zirkoniumdioxids zwischen 40 und 80 Vol.-% liegt,
wobei dem keramischen Material zumindest ein färbendes Oxid aus der Gruppe Pr, Er, Fe, Co, Ni, Ti, V, Cr, Cu, Mn, Tb zugegeben wird.

9. Verfahren nach Anspruch 8,
**dadurch gekennzeichnet,**
**dass** in eine Matrize eine Schicht aus einem ersten keramischen Material einer ersten Zusammensetzung eingebracht wird, dass in der Schicht zumindest eine erste offene Kavität ausgebildet wird, in die ein zweites keramisches Material einer zweiten Zusammensetzung eingebracht wird, und dass die Materialien gemeinsam gepresst und sodann wärmebehandelt werden, wobei vorzugsweise nach Einbringen des zweiten keramischen Materials in dieses eine zweite offene Kavität eingebracht wird, in die ein drittes keramisches Material eingebracht wird, dessen Zusammensetzung von der des ersten und/oder des zweiten keramischen Materials unterschiedlich ist, und dass gegebenenfalls in die Schicht aus dem ersten keramischen Material mehrere erste offene Kavitäten, gegebenenfalls voneinander abweichender Geometrien, ausgebildet werden und in diese das zweite keramische Material eingebracht wird.

10. Verfahren nach zumindest einem der Ansprüche 8 bis 9,
**dadurch gekennzeichnet,**
**dass** nach Einbringen einer ersten Schicht aus einem ersten keramischen Material in eine Matrize die Schicht oberflächlich derart strukturiert wird, dass Erhebungen und von diesen begrenzte Vertiefungen gebildet werden, und sodann in die Matrize ein zweites keramisches Material eingebracht wird, oder dass nach Einbringen der ersten Schicht auf diese eine weitere Schicht aus einer Mischung in die Matrize eingeführt wird, die von der ersten Schicht abweicht, dass die erste Schicht in ihrem Oberflächenbereich mit der zweiten Schicht zur Bildung einer Zwischenschicht durchmischt wird, und dass sodann die zweite Schicht in die Matrize eingebracht wird, wobei vorzugsweise die Struktur durch ein sich relativ zu der ersten Schicht bewegendes, insbesondere drehendes, Element erzeugt wird, das mit einem wellen-, kamm- oder sägezahnartig ausgebildeten Abschnitt die erste Schicht in ihrem Oberflächenbereich strukturiert, oder dass vorzugsweise die Struktur mit einem in Richtung der Oberfläche der ersten Schicht einwirkendem Druckelement erzeugt wird.

11. Zahntechnisches Produkt in Form einer dentalen Restauration, insbesondere Krone, Teilkrone oder Brücke, oder in Form eines Implantatsystems bestehend aus Implantat und Abutment, das vorzugsweise einteilig ausgebildet ist, hergestellt aus einem Rohling nach einem der Ansprüche 1 bis 7,
**dadurch gekennzeichnet,**
**dass** bei einer Restauration diese in Zahnachsenrichtung betrachtet aus zumindest einer wurzelseitig verlaufenden ersten Schicht und einer schneideseitig verlaufenden zweiten Schicht besteht, wobei die Festigkeit der ersten Schicht größer als die der zweiten Schicht und die Transluzenz der zweiten Schicht größer als die der ersten Schicht ist, und/oder dass die Schichten ein erstes färbendes Oxid enthalten, insbesondere zumindest ein Oxid aus der Gruppe Co, Mn, Ni, Cr, vorzugsweise Co₃O₄ oder MnO₂ oder eine Mischung dieser, wobei der Anteil in der ersten Schicht geringer als in der zweiten Schicht ist.

## Claims

1. A blank for use in producing a dental product, such as a dental restoration, veneer or implant component, consisting of or containing zirconium dioxide (ZrO₂) with a tetragonal phase, the zirconium dioxide being stabilized with gadolinium(III) oxide (Gd₂O₃) as the main stabilizer and with Y₂O₃ as the secondary stabilizer,
the zirconium dioxide being stabilized with Gd₂O₃ as the main stabilizer, as well as with the secondary stabilizer with a content between 0.2 and 0.8 mol% relative to the content of ZrO₂, and the tetragonal phase of the zirconium dioxide being between 40 and 80 vol%,
wherein the Gd₂O₃ content is between 2.5 and 4 mol%, preferably between 3 and 4 mol%, relative to the ZrO₂ content.
wherein the blank contains at least one colouring oxide from the group Pr, Er, Fe, Co, Ni, Ti, V, Cr, Cu, Mn, Tb,
wherein the blank has at least two regions with compositions that differ from one another, wherein the total content and/or the stabilizers in the at least two regions are different, wherein the content of Y₂O₃ in the at least two regions is the same,
wherein the content of the colouring oxide or oxides in the at least two regions differs from one another.

2. The blank according to claim 1,
**characterized in that**
the tetragonal phase of the zirconium dioxide is between 45 vol% and 75 vol%.

3. The blank according to at least one of the preceding claims,
**characterized in that**
the proportion of the colouring oxide(s) in wt% of the blank is max. 1.5 wt%.

4. The blank according to at least one of the preceding claims,
**characterized in that**
the blank or a region of the blank contains at least one element that produces a fluorescence effect, in particular at least one oxide from the group Bi, Tb, Tm, Pr, with a proportion in wt% of between 0.005 and 2.0 wt%, preferably between 0.005 and 0.5 wt%.

5. The blank according to at least one of the preceding claims,
**characterized in that**
a first region has a cavity, within which the second region, which has a composition that differs from the first region, extends, wherein the first region optionally has a plurality of cavities, optionally of different internal geometry, in which a plurality of second regions extend, and **in that** preferably the first region has a greater translucency than the second region, the strength of which is greater than that of the first region.

6. The blank according to at least one of the preceding claims,
**characterized in that**
the blank has at least three layers, wherein the middle layer running between an upper and a lower layer consists of a material of the upper layer and the lower layer.

7. The blank according to at least one of the preceding claims,
**characterized in that**
the blank is multi-layered, comprising at least a bottom layer and a top layer of different compositions, wherein the layers contain at least one first colouring oxide from the group Co, Mn, Ni, Cr, the proportion of which in the bottom layer having the first colouring oxide is less than in the top layer having the first colouring oxide, wherein the first colouring oxide is preferably at least one oxide from the group Co, Mn, Ni, Cr, in particular Co₂O₃ or MnO₂ or a mixture thereof.

8. A method for producing a blank intended for producing a dental product, in particular a dental restoration, veneer, implant component, powdered material containing or consisting of stabilized zirconium dioxide being placed in a mold, pressed and then subjected to at least one heat treatment, the zirconium dioxide being mixed with gadolinium(III) oxide (Gd₂O₃) as the main stabilizer and with Y₂O₃ as the secondary stabilizer, and having the tetragonal phase, wherein the ceramic powder used is one whose content of gadolinium(III) oxide (Gd₂O₃) is between 2.5 and 4 mol% and the content of the secondary stabilizer is between 0.2 and 0.8 mol%, in each case relative to the ZrO₂ content, and the tetragonal phase of the zirconium dioxide is between 40 and 80 vol%.,
wherein at least one colouring oxide from the group Pr, Er, Fe, Co, Ni, Ti, V, Cr, Cu, Mn, Tb is added to the ceramic material.

9. The method according to claim 8,
**characterized in that**
a layer of a first ceramic material of a first composition is introduced into a die, **in that** at least a first open cavity is formed in the layer, into which a second ceramic material of a second composition is introduced, and **in that** the materials are pressed together and then heat-treated, wherein preferably after the second ceramic material has been introduced therein, a second open cavity is introduced, into which a third ceramic material is introduced, the composition of which is different from that of the first and/or the second ceramic material, and **in that** optionally a plurality of first open cavities, optionally of different geometries, are formed in the layer of the first ceramic material and the second ceramic material is introduced into these cavities.

10. The method according to at least one of claims 8 to 9,
**characterized in that**
after a first layer of a first ceramic material has been introduced into a die, the layer is structured on the surface in such a way that elevations and depressions bounded by these are formed, and a second ceramic material is subsequently introduced into the die, or **in that**, after the first layer has been introduced onto the latter, a further layer of a mixture that differs from the first layer is introduced into the die, **in that** the first layer is mixed in its surface region with the second layer to form an intermediate layer, and **in that** the second layer is then introduced into the die, wherein the structure is preferably produced by an element that moves, in particular rotates, relative to the first layer and that structures the surface region of the first layer with a wave-like, comb-like or sawtooth-like portion, or **in that** preferably the structure is created with a pressure element acting in the direction of the surface of the first layer.

11. A dental product in the form of a dental restoration, in particular a crown, partial crown or bridge, or in the form of an implant system consisting of an implant and abutment, which is preferably designed in one piece, produced from a blank according to any one of claims 1 to 7, **characterized in that**
in the case of a restoration, viewed in the direction of the tooth axis, it consists of at least a first layer running on the root side and a second layer running on the incisal side, wherein the strength of the first layer is greater than that of the second layer and the translucency of the second layer is greater than that of the first layer, and/or **in that** the layers contain a first colouring oxide, in particular at least one oxide from the group Co, Mn, Ni, Cr, preferably Co₃O₄ or MnO₂ or a mixture thereof, wherein the proportion in the first layer is lower than in the second layer.

## Revendications

1. Plombage blanc destiné à être utilisé dans la production d'un produit dentaire, tel qu'une restauration dentaire, un composant de facette ou d'implant, consistant en ou contenant du dioxyde de zirconium (ZrO₂) avec une phase tétragonale, le dioxyde de zirconium étant stabilisé avec de l'oxyde de gadolinium(III) (Gd₂O₃) comme stabilisant principal et avec de l'Y₂O₃ comme stabilisant secondaire,
le dioxyde de zirconium étant stabilisé avec du Gd₂O₃ comme stabilisant principal, ainsi qu'avec le stabilisant secondaire à une teneur comprise entre 0,2 et 0,8 % molaire par rapport à la teneur en ZrO₂, et la phase tétragonale du dioxyde de zirconium entre 40 et 80 % en volume,
la teneur en Gd₂O₃ étant comprise entre 2,5 et 4 % molaire, de préférence entre 3 et 4 % molaire, par rapport à la teneur en ZrO_{2,}
le plombage blanc contenant au moins un oxyde colorant du groupe Pr, Er, Fe, Co, Ni, Ti, V, Cr, Cu, Mn, Tb,
le plombage blanc présentant au moins deux zones ayant des compositions différentes l'une de l'autre,
la teneur totale et/ou les stabilisants étant différents dans les au moins deux zones, Y₂O₃ étant identique en termes de teneur dans les au moins deux zones,
la teneur en oxyde(s) colorant(s) dans les au moins deux zones étant différente.

2. Plombage blanc selon la revendication 1,
**caractérisé en ce**
**que** la phase tétragonale de l'oxyde de zirconium est comprise entre 45 % en volume et 75 % en volume.

3. Plombage blanc selon au moins une des revendications précédentes,
**caractérisé en ce**
**que** la proportion de l'oxyde colorant ou des oxydes colorants en % massique du plombage blanc est au maximum de 1,5.

4. Plombage blanc selon au moins une des revendications précédentes,
**caractérisé en ce**
**que** le plombage blanc ou une zone du plombage blanc contient au moins un élément produisant un effet de fluorescence, notamment au moins un oxyde du groupe Bi, Tb, Tm, Pr, avec un pourcentage massique compris entre 0,005 et 2,0 %, de préférence entre 0,005 et 0,5 %.

5. Plombage blanc selon au moins une des revendications précédentes,
**caractérisé en ce**
**qu'**une première zone présente une cavité à l'intérieur de laquelle s'étend la deuxième zone, dont la composition diffère de la première zone, la première zone présentant éventuellement une pluralité de cavités, éventuellement de géométrie interne différente, dans lesquelles une pluralité de deuxièmes zones s'étendent, et que de préférence la première zone a une translucidité plus élevée que celle de la deuxième zone, dont la résistance est supérieure à celle de la première zone.

6. Plombage blanc selon au moins une des revendications précédentes,
**caractérisé en ce**
**que** le plombage blanc comporte au moins trois couches, la couche médiane s'étendant entre une couche supérieure et une couche médiane étant constituée d'un matériau de la couche supérieure et de la couche inférieure.

7. Plombage blanc selon au moins une des revendications précédentes,
**caractérisé en ce**
**que** le plombage blanc est formé en plusieurs couches comprenant au moins une couche inférieure et une couche supérieure de compositions différentes, les couches contenant au moins un premier oxyde colorant du groupe Co, Mn, Ni, Cr, dont la proportion dans la couche inférieure présentant le premier oxyde colorant est inférieure à celle de la couche supérieure présentant le premier oxyde colorant, le premier oxyde colorant étant de préférence au moins un oxyde du groupe Co, Mn, Ni, Cr, en particulier Co₂O₃ ou MnO₂ ou leur mélange.

8. Procédé de fabrication d'un plombage blanc destiné à la fabrication d'un produit dentaire, en particulier d'un composant de restauration dentaire, de facette, d'implant, dans lequel un matériau pulvérulent contenant du dioxyde de zirconium stabilisé ou composé de ce dernier est introduit dans un moule, pressé puis soumis à au moins un traitement thermique, le dioxyde de zirconium étant stabilisé avec de l'oxyde de gadolinium(III) (Gd₂O₃) comme stabilisant principal et de l'Y₂O₃ comme stabilisant secondaire et présentant la phase tétragonale,
une poudre dont la teneur en oxyde de gadolinium(III) (Gd₂O₃) est comprise entre 2,5 et 4 % molaire étant utilisée comme poudre céramique, et la teneur en stabilisant secondaire étant comprise entre 0,2 et 0,8 % molaire, dans chaque cas par rapport à la teneur en ZrO₂, et la phase tétragonale du dioxyde de zirconium étant comprise entre 40 et 80 % en volume,
au moins un oxyde colorant du groupe Pr, Er, Fe, Co, Ni, Ti, V, Cr, Cu, Mn, Tb étant ajouté au matériau céramique.

9. Procédé selon la revendication 8,
**caractérisé en ce**
**qu'**une couche d'un premier matériau céramique d'une première composition est introduite dans un moule, qu'au moins une première cavité est formée dans la couche, dans laquelle un second matériau céramique d'une seconde composition est introduit, et que les matériaux sont pressés ensemble puis soumis à un traitement thermique, une deuxième cavité étant creusée de préférence dans le deuxième matériau céramique après introduction de ce dernier, cette cavité étant remplie d'un troisième matériau céramique, dont la composition est différente de celle du premier et/ou du deuxième matériau céramique, et qu'une pluralité de premières cavités, éventuellement avec des géométries différentes, sont éventuellement pratiquées dans la couche, puis remplies avec le deuxième matériau céramique.

10. Procédé selon au moins l'une des revendications 8 à 9,
**caractérisé en ce**
**qu'**après introduction d'une première couche d'un premier matériau céramique dans un moule, la couche est structurée superficiellement de façon à former des reliefs délimitant des creux, puis qu'un second matériau céramique est introduit dans le moule, ou qu'après l'introduction de la première couche, une autre couche d'un mélange est introduite par-dessus dans le moule, qui diffère de la première couche, que la première couche est mélangée à la surface avec la deuxième couche pour former une couche intermédiaire, puis que la deuxième couche est ensuite introduite dans le moule, la structure étant de préférence réalisée par un élément mobile, notamment rotatif, par rapport à la première couche et qui structure la surface de la première couche avec une section ondulée, en peigne ou en dents de scie, ou que la structure est produite de préférence avec un élément de pression agissant en direction de la première couche.

11. Produit dentaire sous forme de restauration dentaire, notamment une couronne, couronne partielle ou pontage, ou sous forme de système implantaire constitué d'un implant et d'un pilier, de préférence en une seule pièce, réalisé à partir d'un plombage blanc selon l'une des revendications 1 à 7,
**caractérisé en ce**
**que** dans le cas d'une restauration, vue dans la direction de l'axe de la dent, elle consiste en au moins une première couche s'étendant du côté de la racine et une seconde couche s'étendant du côté incisif, la résistance de la première couche étant supérieure à celle de la deuxième couche, et la translucidité de la deuxième couche étant supérieure à celle de la première couche, et/ou que les couches contiennent un premier oxyde colorant, notamment au moins un oxyde du groupe Co, Mn, Ni, Cr, de préférence Co₃O₄ ou MnO₂ ou leur mélange la proportion dans la première couche étant plus faible que dans la deuxième couche.
